# EUROPEAN PATENT SPECIFICATION

(11) **EP 1 985 301 B1**
(45) Date of publication and mention of the grant of the patent: **16.11.2016**
(21) Application number: 07706343.6
(22) Date of filing: 16.02.2007
(51) Int. Cl.: A61K 38/00, A61K 39/29, A61P 31/14, A61P 37/04

(54) **METHOD OF INDUCING CYTOTOXIC T-CELL, CYTOTOXIC T-CELL INDUCER AND, PRODUCED THEREWITH, PHARMACEUTICAL COMPOSITION AND VACCINE**
VERFAHREN ZUR INDUKTION EINER ZYTOTOXISCHEN T-ZELLE, INDUKTOR EINER ZYTOTOXISCHEN T-ZELLE UND DAMIT HERGESTELLTE ZUSAMMENSETZUNG BZW. DAMIT HERGESTELLTER IMPFSTOFF
PROCEDE D'INDUCTION DE CELLULE T CYTOTOXIQUE, AGENT INDUISANT DE CELLULE T CYTOTOXIQUE ET, PRODUIT EN COMBINAISON, COMPOSITION PHARMACEUTIQUE ET VACCIN

(30) Priority: 17.02.2006 JP 2006041508
(43) Date of publication of application: 29.10.2008
(60) Divisional application: 12167426.1
(73) Proprietor: NEC Corporation, Minato-ku Tokyo 108-8001 (JP); Kochi University, Kochi-shi Kochi 7808520 (JP); Ehime University, Matsuyama-shi, Ehime 790-8577 (JP)
(72) Inventor: MIYAKAWA, Tomoya, Minato-ku, Tokyo, 108-8001 (JP); UDAKA, Keiko, Nankoku-shi, Kochi, 783-8505 (JP); ONJI, Morikazu, Toon-shi, Ehime, 791-0204 (JP)
(74) Representative: Robson, Aidan John
(86) International application number: PCT/JP2007/000095
(87) International publication number: WO 2007/094137

(56) References cited:
- WO-A1-01/21189
- WO-A1-2005/028503
- WO-A1-2005/105993
- WO-A2-2005/118626
- KUROKOHCHI K ET AL: "A novel cytotoxic T-cell epitope presented by HLA-A24 molecul e in hepatitis C virus infection" JOURNAL OF HEPATOLOGY, MUNKSGAARD INTERNATIONAL PUBLISHERS, COPENHAGEN, DK LNKD- DOI:10.1016/S0168-8278(01)00041-1, vol. 34, no. 6, 1 June 2001 (2001-06-01), pages 930-935, XP002990779 ISSN: 0168-8278
- TAMURA ET AL: "Identification of hepatitis C virus 1b-derived peptides recognized by both cellular and humoral immune systems in HCV1b<+> HLA-A24<+> patients" HEPATOLOGY RESEARCH, AMSTERDAM, NL, vol. 32, no. 4, 1 August 2005 (2005-08-01) , pages 227-234, XP005042313 ISSN: 1386-6346
- WENTWORTH P.A. ET AL.: 'Identification of A2-restricted hepatitis C virus-specific cytotoxic regions of the viral genome' INT. IMMUNOL. vol. 8, no. 5, 1996, pages 651 - 659, XP001027156

### SEQUENCE LISTING

<110> NEC Corporation Kochi University Ehime University
<120> Method for inducing CTL
<130> 95305012
<160> 16
<170> PatentIn version 3.3
<210> 1
   <211> 9
   <212> PRT
   <213> Hepatitis C virus
<400> 1
   Leu Leu Pro Arg Arg Gly Pro Arg Leu
   1               5
<210> 2
   <211> 9
   <212> PRT
   <213> Hepatitis C virus
<400> 2
   Trp His Tyr Pro Cys Thr Val Asn Phe
   1               5
<210> 3
   <211> 9
   <212> PRT
   <213> Hepatitis C virus
<400> 3
   Leu Leu Ser Thr Thr Glu Trp Gln Ile
   1               5
<210> 4
   <211> 9
   <212> PRT
   <213> Hepatitis C virus
<400> 4
   Ile Leu Pro Cys Ser Phe Thr Thr Leu
   1               5
<210> 5
   <211> 9
   <212> PRT
   <213> Hepatitis C virus
<400> 5
   Ala Leu Tyr Gly Val Trp Pro Leu Leu
   1               5
<210> 6
   <211> 9
   <212> PRT
   <213> Hepatitis C virus
<400> 6
   Tyr Tyr Lys Val Phe Leu Ala Arg Leu
   1               5
<210> 7
   <211> 9
   <212> PRT
   <213> Hepatitis C virus
<400> 7
   Val Phe Ser Asp Met Glu Thr Lys Leu
   1               5
<210> 8
   <211> 9
   <212> PRT
   <213> Hepatitis C virus
<400> 8
   Ala Tyr Ser Gln Gln Thr Arg Gly Leu
   1               5
<210> 9
   <211> 9
   <212> PRT
   <213> Hepatitis C virus
<400> 9
   Ile Thr Tyr Ser Thr Tyr Cys Lys Phe
   1               5
<210> 10
   <211> 9
   <212> PRT
   <213> Hepatitis C virus
<400> 10
   Arg Tyr Ala Pro Ala Cys Lys Pro Leu
   1               5
<210> 11
   <211> 9
   <212> PRT
   <213> Hepatitis C virus
<400> 11
   Ser Met Leu Thr Asp Pro Ser His Ile
   1               5
<210> 12
   <211> 9
   <212> PRT
   <213> Hepatitis C virus
<400> 12
   Ser Tyr Thr Trp Thr Gly Ala Leu Ile
   1               5
<210> 13
   <211> 9
   <212> PRT
   <213> Hepatitis C virus
<400> 13
   Ile Leu Met Thr His Phe Phe Ser Ile
   1               5
<210> 14
   <211> 9
   <212> PRT
   <213> Hepatitis C virus
<400> 14
   Phe Trp Ala Lys His Met Trp Asn Phe
   1               5
<210> 15
   <211> 3010
   <212> PRT
   <213> Hepatitis C virus
<400> 15
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Met Gly Trp Ala Gly Trp
   85 90 95
   Leu Leu Ser Pro Arg Gly Ser Arg Pro Ser Trp Gly Pro Thr Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
   180 185 190
   Glu Val Arg Asn Val Ser Gly Ile Tyr His Val Thr Asn Asp Cys Ser
   195 200 205
   Asn Ser Ser Ile Val Tyr Glu Ala Ala Asp Met Ile Met His Thr Pro
   210 215 220
   Gly Cys Val Pro Cys Val Arg Glu Ser Asn Phe Ser Arg Cys Trp Val
   225 230 235 240
   Ala Leu Thr Pro Thr Leu Ala Ala Arg Asn Ser Ser Ile Pro Thr Thr
   245 250 255
   Thr Ile Arg Arg His Val Asp Leu Leu Val Gly Ala Ala Ala Leu Cys
   260 265 270
   Ser Ala Met Tyr Val Gly Asp Leu Cys Gly Ser Val Phe Leu Val Ser
   275 280 285
   Gln Leu Phe Thr Phe Ser Pro Arg Arg Tyr Glu Thr Val Gln Asp Cys
   290 295 300
   Asn Cys Ser Ile Tyr Pro Gly His Val Ser Gly His Arg Met Ala Trp
   305 310 315 320
   Asp Met Met Met Asn Trp Ser Pro Thr Thr Ala Leu Val Val Ser Gln
   325 330 335
   Leu Leu Arg Ile Pro Gln Ala Val Val Asp Met Val Ala Gly Ala His
   340 345 350
   Trp Gly Val Leu Ala Gly Leu Ala Tyr Tyr Ser Met Val Gly Asn Trp
   355 360 365
   Ala Lys Val Leu Ile Val Met Leu Leu Phe Ala Gly Val Asp Gly His
   370 375 380
   Thr His Val Thr Gly Gly Arg Val Ala Ser Ser Thr Gln Ser Leu Val
   385 390 395 400
   Ser Trp Leu Ser Gln Gly Pro Ser Gln Lys Ile Gln Leu Val Asn Thr
   405 410 415
   Asn Gly Ser Trp His Ile Asn Arg Thr Ala Leu Asn Cys Asn Asp Ser
   420 425 430
   Leu Gln Thr Gly Phe Ile Ala Ala Leu Phe Tyr Ala His Arg Phe Asn
   435 440 445
   Ala Ser Gly Cys Pro Glu Arg Met Ala Ser Cys Arg Pro Ile Asp Glu
   450 455 460
   Phe Ala Gln Gly Trp Gly Pro Ile Thr His Asp Met Pro Glu Ser Ser
   465 470 475 480
   Asp Gln Arg Pro Tyr Cys Trp His Tyr Ala Pro Arg Pro Cys Gly Ile
   485 490 495
   Val Pro Ala Ser Gln Val Cys Gly Pro Val Tyr Cys Phe Thr Pro Ser
   500 505 510
   Pro Val Val Val Gly Thr Thr Asp Arg Phe Gly Ala Pro Thr Tyr Ser
   515 520 525
   Trp Gly Glu Asn Glu Thr Asp Val Leu Leu Leu Ser Asn Thr Arg Pro
   530 535 540
   Pro Gln Gly Asn Trp Phe Gly Cys Thr Trp Met Asn Ser Thr Gly Phe
   545 550 555 560
   Thr Lys Thr Cys Gly Gly Pro Pro Cys Asn Ile Gly Gly Val Gly Asn
   565 570 575
   Asn Thr Leu Val Cys Pro Thr Asp Cys Phe Arg Lys His Pro Glu Ala
   580 585 590
   Thr Tyr Thr Lys Cys Gly Ser Gly Pro Trp Leu Thr Pro Arg Cys Met
   595 600 605
   Val Asp Tyr Pro Tyr Arg Leu Trp His Tyr Pro Cys Thr Val Asn Phe
   610 615 620
   Thr Val Phe Lys Val Arg Met Tyr Val Gly Gly Val Glu His Arg Leu
   625 630 635 640
   Asn Ala Ala Cys Asn Trp Thr Arg Gly Glu Arg Cys Asp Leu Glu Asp
   645 650 655
   Arg Asp Arg Ser Glu Leu Ser Pro Leu Leu Leu Ser Thr Thr Glu Trp
   660 665 670
   Gln Ile Leu Pro Cys Ser Phe Thr Thr Leu Pro Ala Leu Ser Thr Gly
   675 680 685
   Leu Ile His Leu His Arg Asn Ile Val Asp Val Gln Tyr Leu Tyr Gly
   690 695 700
   Ile Gly Ser Ala Val Val Ser Phe Ala Ile Lys Trp Glu Tyr Ile Leu
   705 710 715 720
   Leu Leu Phe Leu Leu Leu Ala Asp Ala Arg Val Cys Ala Cys Leu Trp
   725 730 735
   Met Met Leu Leu Ile Ala Gln Ala Glu Ala Thr Leu Glu Asn Leu Val
   740 745 750
   Val Leu Asn Ala Ala Ser Val Ala Gly Ala His Gly Leu Leu Ser Phe
   755 760 765
   Leu Val Phe Phe Cys Ala Ala Trp Tyr Ile Lys Gly Arg Leu Val Pro
   770 775 780
   Gly Ala Ala Tyr Ala Leu Tyr Gly Val Trp Pro Leu Leu Leu Leu Leu
   785 790 795 800
   Leu Ala Leu Pro Pro Arg Ala Tyr Ala Met Asp Arg Glu Met Ala Ala
   805 810 815
   Ser Cys Gly Gly Ala Val Phe Val Gly Leu Val Leu Leu Thr Leu Ser
   820 825 830
   Pro Tyr Tyr Lys Val Phe Leu Ala Arg Leu Ile Trp Trp Leu Gln Tyr
   835 840 845
   Phe Ile Thr Arg Ala Glu Ala His Leu Gln Val Trp Val Pro Pro Leu
   850 855 860
   Asn Val Arg Gly Gly Arg Asp Ala Ile Ile Leu Leu Thr Cys Ala Val
   865 870 875 880
   His Pro Glu Leu Ile Phe Asp Ile Thr Lys Leu Leu Leu Ala Ile Leu
   885 890 895
   Gly Pro Leu Met Val Leu Gln Ala Gly Ile Thr Arg Val Pro Tyr Phe
   900 905 910
   Val Arg Ala Gln Gly Leu Ile Arg Ala Cys Met Leu Val Arg Lys Val
   915 920 925
   Ala Gly Gly His Tyr Val Gln Met Ala Phe Met Lys Leu Ala Ala Leu
   930 935 940
   Thr Gly Thr Tyr Val Tyr Asp His Leu Thr Pro Leu Arg Asp Trp Ala
   945 950 955 960
   His Ala Gly Leu Arg Asp Leu Ala Val Ala Val Glu Pro Val Val Phe
   965 970 975
   Ser Asp Met Glu Thr Lys Leu Ile Thr Trp Gly Ala Asp Thr Ala Ala
   980 985 990
   Cys Gly Asp Ile Ile Ser Gly Leu Pro Val Ser Ala Arg Arg Gly Lys
   995 1000 1005
   Glu Ile Leu Leu Gly Pro Ala Asp Ser Phe Gly Glu Gln Gly Trp
   1010 1015 1020
   Arg Leu Leu Ala Pro Ile Thr Ala Tyr Ser Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Asp Gly Glu Val Gln Val Leu Ser Thr Ala Thr Gln Ser
   1055 1060 1065
   Phe Leu Ala Thr Cys Val Asn Gly Val Cys Trp Thr Val Tyr His
   1070 1075 1080
   Gly Ala Gly Ser Lys Thr Leu Ala Gly Pro Lys Gly Pro Ile Thr
   1085 1090 1095
   Gln Met Tyr Thr Asn Val Asp Gln Asp Leu Val Gly Trp Pro Ala
   1100 1105 1110
   Pro Pro Gly Ala Arg Ser Met Thr Pro Cys Thr Cys Gly Ser Ser
   1115 1120 1125
   Asp Leu Tyr Leu Val Thr Arg His Ala Asp Val Val Pro Val Arg
   1130 1135 1140
   Arg Arg Gly Asp Ser Arg Gly Ser Leu Leu Ser Pro Arg Pro Ile
   1145 1150 1155
   Ser Tyr Leu Lys Gly Ser Ser Gly Gly Pro Leu Leu Cys Pro Ser
   1160 1165 1170
   Gly His Val Val Gly Ile Phe Arg Ala Ala Val Cys Thr Arg Gly
   1175 1180 1185
   Val Ala Lys Ala Val Asp Phe Ile Pro Val Glu Ser Met Glu Thr
   1190 1195 1200
   Thr Met Arg Ser Pro Val Phe Thr Asp Asn Ser Ser Pro Pro Ala
   1205 1210 1215
   Val Pro Gln Thr Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Lys Val Leu Val Leu Asn Pro Ser Val Ala Ala Thr Leu Gly
   1250 1255 1260
   Phe Gly Ala Tyr Met Ser Lys Ala His Gly Ile Glu Pro Asn Ile
   1265 1270 1275
   Arg Thr Gly Val Arg Thr Ile Thr Thr Gly Gly Pro Ile Thr Tyr
   1280 1285 1290
   Ser Thr Tyr Cys Lys Phe Leu Ala Asp Gly Gly Cys Ser Gly Gly
   1295 1300 1305
   Ala Tyr Asp Ile Ile Ile Cys Asp Glu Cys His Ser Thr Asp Ser
   1310 1315 1320
   Thr Thr Ile Leu Gly Ile Gly Thr Val Leu Asp Gln Ala Glu Thr
   1325 1330 1335
   Ala Gly Ala Arg Leu Val Val Leu Ala Thr Ala Thr Pro Pro Gly
   1340 1345 1350
   Ser Ile Thr Val Pro His Pro Asn Ile Glu Glu Val Ala Leu Ser
   1355 1360 1365
   Asn Thr Gly Glu Ile Pro Phe Tyr Gly Lys Ala Ile Pro Ile Glu
   1370 1375 1380
   Ala Ile Lys Gly Gly Arg His Leu Ile Phe Cys His Ser Lys Lys
   1385 1390 1395
   Lys Cys Asp Glu Leu Ala Ala Lys Leu Thr Gly Leu Gly Leu Asn
   1400 1405 1410
   Ala Val Ala Tyr Tyr Arg Gly Leu Asp Val Ser Val Ile Pro Thr
   1415 1420 1425
   Ser Gly Asp Val Val Val Val Ala Thr Asp Ala Leu Met Thr Gly
   1430 1435 1440
   Phe Thr Gly Asp Phe Asp Ser Val Ile Asp Cys Asn Thr Cys Val
   1445 1450 1455
   Thr Gln Thr Val Asp Phe Ser Leu Asp Pro Thr Phe Thr Ile Glu
   1460 1465 1470
   Thr Thr Thr Leu Pro Gln Asp Ala Val Ser Arg Ala Gln Arg Arg
   1475 1480 1485
   Gly Arg Thr Gly Arg Gly Arg Ser Gly Ile Tyr Arg Phe Val Thr
   1490 1495 1500
   Pro Gly Glu Arg Pro Ser Gly Met Phe Asp Ser Ser Val Leu Cys
   1505 1510 1515
   Glu Cys Tyr Asp Ala Gly Cys Ala Trp Tyr Glu Leu Thr Pro Ala
   1520 1525 1530
   Glu Thr Ser Val Arg Leu Arg Ala Tyr Leu Asn Thr Pro Gly Leu
   1535 1540 1545
   Pro Val Cys Gln Asp His Leu Glu Phe Trp Glu Ser Val Phe Thr
   1550 1555 1560
   Gly Leu Thr His Ile Asp Ala His Phe Leu Ser Gln Thr Lys Gln
   1565 1570 1575
   Ala Gly Asp Asn Leu Pro Tyr Leu Val Ala Tyr Gln Ala Thr Val
   1580 1585 1590
   Cys Ala Arg Ala Gln Ala Pro Pro Pro Ser Trp Asp Gln Met Trp
   1595 1600 1605
   Lys Cys Leu Ile Arg Leu Lys Pro Thr Leu His Gly Pro Thr Pro
   1610 1615 1620
   Leu Leu Tyr Arg Leu Gly Ala Val Gln Asn Glu Val Thr Leu Thr
   1625 1630 1635
   His Pro Ile Thr Lys Tyr Ile Met Ala Cys Met Ser Ala Asp Leu
   1640 1645 1650
   Glu Val Val Thr Ser Thr Trp Val Leu Val Gly Gly Val Leu Ala
   1655 1660 1665
   Ala Leu Ala Ala Tyr Cys Leu Thr Thr Gly Ser Val Val Ile Val
   1670 1675 1680
   Gly Arg Ile Ile Leu Ser Gly Arg Pro Ala Val Ile Pro Asp Arg
   1685 1690 1695
   Glu Val Leu Tyr Gln Glu Phe Asp Glu Met Glu Glu Cys Ala Ser
   1700 1705 1710
   His Leu Pro Tyr Ile Glu Gln Gly Met Gln Leu Ala Glu Gln Phe
   1715 1720 1725
   Lys Gln Lys Ala Leu Gly Leu Leu Gln Thr Ala Thr Lys Gln Ala
   1730 1735 1740
   Glu Ala Ala Ala Pro Val Val Glu Ser Lys Trp Arg Ala Leu Glu
   1745 1750 1755
   Val Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ser Ile Thr Ser Pro Leu Thr Thr
   1790 1795 1800
   Gln Asn Thr Leu Leu Phe Asn Ile Leu Gly Gly Trp Val Ala Ala
   1805 1810 1815
   Gln Leu Ala Pro Pro Ser Ala Ala Ser Ala Phe Val Gly Ala Gly
   1820 1825 1830
   Ile Ala Gly Ala Ala Val Gly Ser Ile Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Val Met Ser Gly Glu Met Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Gln Ile Leu Ser Ser Leu Thr Ile
   1940 1945 1950
   Thr Gln Leu Leu Lys Arg Leu His Gln Trp Ile Asn Glu Asp Cys
   1955 1960 1965
   Ser Thr Pro Cys Ser Gly Ser Trp Leu Lys Asp Val Trp Asp Trp
   1970 1975 1980
   Ile Cys Thr Val Leu Ser Asp Phe Lys Thr Trp Leu Gln Ser Lys
   1985 1990 1995
   Leu Leu Pro Arg Leu Pro Gly Leu Pro Phe Leu Ser Cys Gln Arg
   2000 2005 2010
   Gly Tyr Lys Gly Val Trp Arg Gly Asp Gly Ile Met Gln Thr Thr
   2015 2020 2025
   Cys Pro Cys Gly Ala Gln Ile Thr Gly His Val Lys Asn Gly Ser
   2030 2035 2040
   Met Arg Ile Val Gly Pro Lys Thr Cys Ser Asn Thr Trp His Gly
   2045 2050 2055
   Thr Phe Pro Ile Asn Ala Tyr Thr Thr Gly Pro Cys Thr Pro Ser
   2060 2065 2070
   Pro Ala Pro Asn Tyr Ser Arg Ala Leu Trp Arg Val Ala Ala Glu
   2075 2080 2085
   Glu Tyr Val Glu Val Thr Arg Val Gly Asp Phe His Tyr Val Thr
   2090 2095 2100
   Gly Met Thr Thr Asp Asn Val Lys Cys Pro Cys Gln Val Pro Ala
   2105 2110 2115
   Pro Glu Phe Phe Thr Glu Val Asp Gly Val Arg Leu His Arg Tyr
   2120 2125 2130
   Ala Pro Val Cys Lys Pro Leu Leu Arg Glu Glu Val Val Phe Gln
   2135 2140 2145
   Val Gly Leu Asn Gln Tyr Leu Val Gly Ser Gln Leu Pro Cys Glu
   2150 2155 2160
   Pro Glu Pro Asp Val Ala Val Leu Thr Ser Met Leu Thr Asp Pro
   2165 2170 2175
   Ser His Ile Thr Ala Glu Thr Ala Lys Arg Arg Leu Ala Arg Gly
   2180 2185 2190
   Ser Pro Pro Ser Leu Ala Ser Ser Ser Ala Ser Gln Leu Ser Ala
   2195 2200 2205
   Pro Ser Leu Lys Ala Thr Cys Thr Thr His His Asp Ser Pro Asp
   2210 2215 2220
   Ala Asp Leu Ile Glu Ala Asn Leu Leu Trp Arg Gln Glu Met Gly
   2225 2230 2235
   Gly Asn Ile Thr Arg Val Glu Ser Glu Asn Lys Val Val Ile Leu
   2240 2245 2250
   Asp Ser Phe Asp Pro Ile Arg Ala Val Glu Asp Glu Arg Glu Ile
   2255 2260 2265
   Ser Val Pro Ala Glu Ile Leu Arg Lys Pro Arg Lys Phe Pro Pro
   2270 2275 2280
   Ala Leu Pro Ile Trp Ala Arg Pro Asp Tyr Asn Pro Pro Leu Leu
   2285 2290 2295
   Glu Ser Trp Lys Asp Pro Asp Tyr Val Pro Pro Val Val His Gly
   2300 2305 2310
   Cys Pro Leu Pro Ser Thr Lys Ala Pro Pro Ile Pro Pro Pro Arg
   2315 2320 2325
   Arg Lys Arg Thr Val Val Leu Thr Glu Ser Thr Val Ser Ser Ala
   2330 2335 2340
   Leu Ala Glu Leu Ala Thr Lys Thr Phe Gly Ser Ser Gly Ser Ser
   2345 2350 2355
   Ala Val Asp Ser Gly Thr Ala Thr Gly Pro Pro Asp Gln Ala Ser
   2360 2365 2370
   Asp Asp Gly Asp Lys Gly Ser Asp Val Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Gly Glu Ala Gly Glu Asp Val Val Cys
   2405 2410 2415
   Cys Ser Met Ser Tyr Thr Trp Thr Gly Ala Leu Ile Thr Pro Cys
   2420 2425 2430
   Ala Ala Glu Glu Ser Lys Leu Pro Ile Asn Pro Leu Ser Asn Ser
   2435 2440 2445
   Leu Leu Arg His His Ser Met Val Tyr Ser Thr Thr Ser Arg Ser
   2450 2455 2460
   Ala Ser Leu Arg Gln Lys Lys Val Thr Phe Asp Arg Leu Gln Val
       2465                2470                2475
   Leu Asp Asp His Tyr Arg Asp Val Leu Lys Glu Met Lys Ala Lys
       2480                2485                2490
   Ala Ser Thr Val Lys Ala Arg Leu Leu Ser Ile Glu Glu Ala Cys
       2495                2500                2505
   Lys Leu Thr Pro Pro His Ser Ala Lys Ser Lys Phe Gly Tyr Gly
       2510                2515                2520
   Ala Lys Asp Val Arg Ser Leu Ser Ser Arg Ala Val Asn His Ile
       2525                2530                2535
   Arg Ser Val Trp Glu Asp Leu Leu Glu Asp Thr Glu Thr Pro Ile
       2540                2545                2550
   Asp Thr Thr Ile Met Ala Lys Asn Glu Val Phe Cys Val Gln Pro
       2555                2560                2565
   Glu Lys Gly Gly Arg Lys Pro Ala Arg Leu Ile Val Phe Pro Asp
       2570                2575                2580
   Leu Gly Val Arg Val Cys Glu Lys Met Ala Leu Tyr Asp Val Val
       2585                2590                2595
   Ser Thr Leu Pro Gln Ala Val Met Gly Pro Ser Tyr Gly Phe Gln
       2600                2605                2610
   Tyr Ser Pro Gly Gln Arg Val Glu Phe Leu Val Asn Thr Trp Lys
       2615                2620                2625
   Ser Lys Lys Cys Pro Met Gly Phe Ser Tyr Asp Thr Arg Cys Phe
       2630                2635                2640
   Asp Ser Thr Val Thr Glu Asn Asp Ile Arg Thr Glu Glu Ser Ile
       2645                2650                2655
   Tyr Gln Cys Cys Asp Leu Ala Pro Glu Ala Arg Gln Ala Ile Arg
       2660                2665                2670
   Ser Leu Thr Glu Arg Leu Tyr Val Gly Gly Pro Leu Thr Asn Ser
       2675                2680                2685
   Lys Gly Gln Asn Cys Gly Tyr Arg Arg Cys Arg Ala Ser Gly Val
       2690                2695                2700
   Leu Thr Thr Ser Cys Gly Asn Thr Leu Thr Cys Tyr Leu Lys Ala
       2705                2710                2715
   Thr Ala Ala Cys Arg Ala Ala Lys Leu Gln Asp Cys Thr Met Leu
   2720 2725 2730
   Val Asn Gly Asp Asp Leu Val Val Ile Cys Glu Ser Ala Gly Thr
   2735 2740 2745
   Gln Glu Asp Ala Ala Ala Leu Arg Ala Phe Thr Glu Ala Met Thr
   2750 2755 2760
   Arg Tyr Ser Ala Pro Pro Gly Asp Pro Pro Gln Pro Glu Tyr Asp
   2765 2770 2775
   Leu Glu Leu Ile Thr Ser Cys Ser Ser Asn Val Ser Val Ala His
   2780 2785 2790
   Asp Ala Ser Gly Lys Arg Val Tyr Tyr Leu Thr Arg Asp Pro Thr
   2795 2800 2805
   Thr Pro Leu Ala Arg Ala Ala Trp Glu Thr Val Arg His Thr Pro
   2810 2815 2820
   Val Asn Ser Trp Leu Gly Asn Ile Ile Met Tyr Ala Pro Thr Leu
   2825 2830 2835
   Trp Ala Arg Met Ile Leu Met Thr His Phe Phe Ser Ile Leu Leu
   2840 2845 2850
   Ala Gln Glu Gln Leu Glu Lys Ala Leu Asp Cys Gln Ile Tyr Gly
   2855 2860 2865
   Ala Cys Tyr Ser Ile Glu Pro Leu Asp Leu Pro Gln Ile Ile Glu
   2870 2875 2880
   Arg Leu His Gly Leu Ser Ala Phe Ser Leu His Ser Tyr Ser Pro
   2885 2890 2895
   Gly Glu Ile Asn Arg Val Ala Ser Cys Leu Arg Lys Leu Gly Val
   2900 2905 2910
   Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr
   2930 2935 2940
   Leu Phe Asn Trp Ala Val Lys Thr Lys Leu Lys Leu Thr Pro Ile
   2945 2950 2955
   Pro Ala Ala Ser Gln Leu Asp Leu Ser Gly Trp Phe Val Ala Gly
   2960 2965 2970
   Tyr Asn Gly Gly Asp Ile Tyr His Ser Leu Ser Arg Ala Arg Pro
   2975 2980 2985
   Arg Trp Phe Met Leu Cys Leu Leu Leu Leu Ser Val Gly Val Gly
   2990 2995 3000
   Ile Tyr Leu Leu Pro Asn Arg
   3005 3010
<210> 16
   <211> 3010
   <212> PRT
   <213> Hepatitis C virus
<400> 16
   Met Ser Thr Asn Pro Lys Pro Gln Arg Lys Thr Lys Arg Asn Thr Asn
   1 5 10 15
   Arg Arg Pro Gln Asp Val Lys Phe Pro Gly Gly Gly Gln Ile Val Gly
   20 25 30
   Gly Val Tyr Leu Leu Pro Arg Arg Gly Pro Arg Leu Gly Val Arg Ala
   35 40 45
   Thr Arg Lys Thr Ser Glu Arg Ser Gln Pro Arg Gly Arg Arg Gln Pro
   50 55 60
   Ile Pro Lys Ala Arg Arg Pro Glu Gly Arg Thr Trp Ala Gln Pro Gly
   65 70 75 80
   Tyr Pro Trp Pro Leu Tyr Gly Asn Glu Gly Leu Gly Trp Ala Gly Trp
   85 90 95
   Leu Leu Ser Pro Arg Gly Ser Arg Pro Ser Trp Gly Pro Asn Asp Pro
   100 105 110
   Arg Arg Arg Ser Arg Asn Leu Gly Lys Val Ile Asp Thr Leu Thr Cys
   115 120 125
   Gly Phe Ala Asp Leu Met Gly Tyr Ile Pro Leu Val Gly Ala Pro Leu
   130 135 140
   Gly Gly Ala Ala Arg Ala Leu Ala His Gly Val Arg Val Leu Glu Asp
   145 150 155 160
   Gly Val Asn Tyr Ala Thr Gly Asn Leu Pro Gly Cys Ser Phe Ser Ile
   165 170 175
   Phe Leu Leu Ala Leu Leu Ser Cys Leu Thr Ile Pro Ala Ser Ala Tyr
   180 185 190
   Glu Val Arg Asn Val Ser Gly Ile Tyr His Val Thr Asn Asp Cys Ser
   195 200 205
   Asn Ser Ser Ile Val Tyr Glu Ala Ala Asp Val Ile Met His Ala Pro
       210                 215                 220
   Gly Cys Val Pro Cys Val Arg Glu Asn Asn Ser Ser Arg Cys Trp Val
   225                 230                 235                 240
   Ala Leu Thr Pro Thr Leu Ala Ala Arg Asn Ala Ser Val Pro Thr Thr
                   245                 250                 255
   Thr Leu Arg Arg His Val Asp Leu Leu Val Gly Thr Ala Ala Phe Cys
               260                 265                 270
   Ser Ala Met Tyr Val Gly Asp Leu Cys Gly Ser Val Phe Leu Ile Ser
           275                 280                 285
   Gln Leu Phe Thr Phe Ser Pro Arg Arg His Glu Thr Val Gln Asp Cys
       290                 295                 300
   Asn Cys Ser Ile Tyr Pro Gly His Val Ser Gly His Arg Met Ala Trp
   305                 310                 315                 320
   Asp Met Met Met Asn Trp Ser Pro Thr Ala Ala Leu Val Val Ser Gln
                   325                 330                 335
   Leu Leu Arg Ile Pro Gln Ala Val Met Asp Met Val Ala Gly Ala His
               340                 345                 350
   Trp Gly Val Leu Ala Gly Leu Ala Tyr Tyr Ser Met Val Gly Asn Trp
           355                 360                 365
   Ala Lys Val Leu Ile Val Met Leu Leu Phe Ala Gly Val Asp Gly His
       370                 375                 380
   Thr Arg Val Thr Gly Gly Val Gln Gly His Val Thr Ser Thr Leu Thr
   385                 390                 395                 400
   Ser Leu Phe Arg Pro Gly Ala Ser Gln Lys Ile Gln Leu Val Asn Thr
                   405                 410                 415
   Asn Gly Ser Trp His Ile Asn Arg Thr Ala Leu Asn Cys Asn Asp Ser
               420                 425                 430
   Leu Lys Thr Gly Phe Leu Ala Ala Leu Phe Tyr Thr His Lys Phe Asn
           435                 440                 445
   Ala Ser Gly Cys Pro Glu Arg Met Ala Ser Cys Arg Ser Ile Asp Lys
       450                 455                 460
   Phe Asp Gln Gly Trp Gly Pro Ile Thr Tyr Ala Gln Pro Asp Asn Ser
   465                 470                 475                 480
   Asp Gln Arg Pro Tyr Cys Trp His Tyr Ala Pro Arg Gln Cys Gly Ile
                   485                 490                 495
   Val Pro Ala Ser Gln Val Cys Gly Pro Val Tyr Cys Phe Thr Pro Ser
               500                 505                 510
   Pro Val Val Val Gly Thr Thr Asp Arg Phe Gly Ala Pro Thr Tyr Asn
           515                 520                 525
   Trp Gly Asp Asn Glu Thr Asp Val Leu Leu Leu Asn Asn Thr Arg Pro
       530                 535                 540
   Pro His Gly Asn Trp Phe Gly Cys Thr Trp Met Asn Ser Thr Gly Phe
   545                 550                 555                 560
   Thr Lys Thr Cys Gly Gly Pro Pro Cys Asn Ile Arg Gly Val Gly Asn
                   565                 570                 575
   Asn Thr Leu Thr Cys Pro Thr Asp Cys Phe Arg Lys His Pro Asp Ala
               580                 585                 590
   Thr Tyr Thr Lys Cys Gly Ser Gly Pro Trp Leu Thr Pro Arg Cys Leu
           595                 600                 605
   Val Asp Tyr Pro Tyr Arg Leu Trp His Tyr Pro Cys Thr Val Asn Phe
       610                 615                 620
   Thr Ile Phe Lys Val Arg Met Tyr Val Gly Gly Val Glu His Arg Leu
   625                 630                 635                 640
   Asp Ala Ala Cys Asn Trp Thr Arg Gly Glu Arg Cys Asp Leu Glu Asp
                   645                 650                 655
   Arg Asp Arg Ala Glu Leu Ser Pro Leu Leu Leu Ser Thr Thr Glu Trp
               660                 665                 670
   Gln Ile Leu Pro Cys Ser Tyr Thr Thr Leu Pro Ala Leu Ser Thr Gly
           675                 680                 685
   Leu Ile His Leu His Gln Asn Ile Val Asp Ile Gln Tyr Leu Tyr Gly
       690                 695                 700
   Ile Gly Ser Ala Val Val Ser Ile Ala Ile Lys Trp Glu Tyr Val Val
   705                 710                 715                 720
   Leu Leu Phe Leu Leu Leu Ala Asp Ala Arg Val Cys Ala Cys Leu Trp
                   725                 730                 735
   Met Met Leu Leu Ile Ala Gln Ala Glu Ala Ala Leu Glu Asn Leu Val
               740                 745                 750
   Val Leu Asn Ala Ala Ser Val Val Gly Ala His Gly Met Leu Pro Phe
   755 760 765
   Phe Met Phe Phe Cys Ala Ala Trp Tyr Met Lys Gly Arg Leu Val Pro
   770 775 780
   Gly Ala Ala Tyr Ala Phe Tyr Gly Val Trp Pro Leu Leu Leu Leu Leu
   785 790 795 800
   Leu Ala Leu Pro Pro Arg Ala Tyr Ala Met Asp Arg Glu Met Val Ala
   805 810 815
   Ser Cys Gly Gly Gly Val Phe Val Gly Leu Ala Leu Leu Thr Leu Ser
   820 825 830
   Pro Tyr Cys Lys Val Phe Leu Ala Arg Leu Ile Trp Trp Leu Gln Tyr
   835 840 845
   Phe Ile Thr Lys Ala Glu Ala His Leu Gln Val Ser Leu Pro Pro Leu
   850 855 860
   Asn Val Arg Gly Gly Arg Asp Ala Ile Ile Leu Leu Met Cys Ala Val
   865 870 875 880
   His Pro Glu Leu Ile Phe Asp Ile Thr Lys Leu Leu Leu Ser Ile Leu
   885 890 895
   Gly Pro Leu Met Val Leu Gln Ala Ser Leu Ile Arg Val Pro Tyr Phe
   900 905 910
   Val Arg Ala Gln Gly Leu Ile Arg Ala Cys Met Leu Val Arg Lys Ala
   915 920 925
   Ala Gly Gly His Tyr Val Gln Met Ala Phe Val Lys Leu Ala Ala Leu
   930 935 940
   Thr Gly Thr Tyr Val Tyr Asp His Leu Thr Pro Leu Gln Asp Trp Ala
   945 950 955 960
   His Val Gly Leu Arg Asp Leu Ala Val Ala Val Glu Pro Val Val Phe
   965 970 975
   Ser Ala Met Glu Thr Lys Val Ile Thr Trp Gly Ala Asp Thr Ala Ala
   980 985 990
   Cys Gly Asp Ile Ile Ser Gly Leu Pro Val Ser Ala Arg Arg Gly Lys
   995 1000 1005
   Glu Ile Leu Leu Gly Pro Ala Asp Ser Phe Glu Gly Gln Gly Trp
   1010 1015 1020
   Arg Leu Leu Ala Pro Ile Thr Ala Tyr Ser Gln Gln Thr Arg Gly
   1025 1030 1035
   Leu Leu Gly Cys Ile Ile Thr Ser Leu Thr Gly Arg Asp Lys Asn
   1040 1045 1050
   Gln Val Glu Gly Glu Val Gln Val Val Ser Thr Ala Lys Gln Ser
   1055 1060 1065
   Phe Leu Ala Thr Cys Val Asn Gly Ala Cys Trp Thr Val Phe His
   1070 1075 1080
   Gly Ala Gly Ser Lys Thr Leu Ala Ala Ala Lys Gly Pro Ile Thr
   1085 1090 1095
   Gln Met Tyr Thr Asn Val Asp Gln Asp Leu Val Gly Trp Pro Ala
   1100 1105 1110
   Pro Pro Gly Ala Arg Ser Leu Thr Pro Cys Thr Cys Gly Ser Ser
   1115 1120 1125
   Asp Leu Tyr Leu Val Thr Arg His Ala Asp Val Ile Pro Val Arg
   1130 1135 1140
   Arg Arg Gly Asp Ser Arg Gly Ser Leu Leu Ser Pro Arg Pro Ile
   1145 1150 1155
   Ser Tyr Leu Lys Gly Ser Ser Gly Gly Pro Leu Leu Cys Pro Ser
   1160 1165 1170
   Gly His Val Val Gly Ile Phe Arg Ala Ala Val Cys Thr Arg Gly
   1175 1180 1185
   Val Ala Lys Ala Val Asp Phe Ile Pro Val Glu Ser Met Glu Thr
   1190 1195 1200
   Thr Met Arg Ser Pro Val Phe Thr Asp Asn Ser Thr Pro Pro Ala
   1205 1210 1215
   Val Pro Gln Thr Phe Gln Val Ala His Leu His Ala Pro Thr Gly
   1220 1225 1230
   Ser Gly Lys Ser Thr Lys Val Pro Ala Ala Tyr Ala Ala Gln Gly
   1235 1240 1245
   Tyr Met Val Leu Val Leu Asn Pro Ser Val Ala Ala Thr Leu Gly
   1250 1255 1260
   Phe Gly Ala Tyr Met Ser Lys Ala His Gly Ile Asp Pro Asn Ile
   1265 1270 1275
   Arg Thr Gly Val Arg Thr Ile Thr Thr Gly Ala Pro Ile Thr Tyr
   1280 1285 1290
   Ser Thr Tyr Gly Lys Phe Leu Ala Asp Gly Gly Cys Ser Gly Gly
   1295 1300 1305
   Ala Tyr Asp Ile Ile Ile Cys Asp Glu Cys His Ser Thr Asp Ser
   1310 1315 1320
   Thr Ser Ile Leu Gly Ile Gly Thr Val Leu Asp Gln Ala Glu Thr
   1325 1330 1335
   Val Gly Ala Arg Phe Val Val Leu Ala Thr Ala Thr Pro Pro Gly
   1340 1345 1350
   Ser Ile Thr Phe Pro His Pro Asn Ile Glu Glu Val Pro Leu Ala
   1355 1360 1365
   Asn Thr Gly Glu Ile Pro Phe Tyr Ala Lys Thr Ile Pro Ile Glu
   1370 1375 1380
   Val Ile Arg Gly Gly Arg His Leu Ile Phe Cys His Ser Lys Lys
   1385 1390 1395
   Lys Cys Asp Glu Leu Pro Ala Lys Leu Ser Ala Leu Gly Leu Asn
   1400 1405 1410
   Ala Val Ala Tyr Tyr Arg Gly Leu Asp Val Ser Val Ile Pro Ala
   1415 1420 1425
   Ser Gly Asp Val Val Val Val Ala Thr Asp Ala Leu Met Thr Gly
   1430 1435 1440
   Phe Thr Gly Asp Phe Asp Ser Val Ile Asp Cys Asn Thr Cys Val
   1445 1450 1455
   Thr Gln Thr Val Asp Phe Ser Leu Asp Pro Thr Phe Thr Ile Glu
   1460 1465 1470
   Thr Thr Thr Val Pro Gln Asp Ala Val Ser Arg Thr Gln Arg Arg
   1475 1480 1485
   Gly Arg Thr Gly Arg Gly Arg Arg Gly Ile Tyr Arg Phe Val Thr
   1490 1495 1500
   Pro Gly Glu Arg Pro Ser Ala Met Phe Asp Ser Ser Val Leu Cys
   1505 1510 1515
   Glu Cys Tyr Asp Ala Gly Cys Ala Trp Tyr Glu Leu Thr Pro Ala
   1520 1525 1530
   Glu Thr Ser Val Arg Leu Arg Ala Tyr Leu Asn Thr Pro Gly Leu
   1535 1540 1545
   Pro Val Cys Gln Asp His Leu Glu Phe Trp Glu Ser Val Phe Thr
   1550 1555 1560
   Gly Leu Thr His Ile Asp Ala His Phe Leu Ser Gln Thr Lys Gln
   1565 1570 1575
   Ala Gly Asp Asn Phe Pro Tyr Leu Val Ala Tyr Gln Ala Thr Val
   1580 1585 1590
   Cys Ala Arg Ala Lys Ala Pro Pro Pro Ser Trp Asp Gln Met Trp
   1595 1600 1605
   Lys Cys Leu Ile Arg Leu Lys Pro Thr Leu His Gly Pro Thr Pro
   1610 1615 1620
   Leu Leu Tyr Arg Leu Gly Ala Val Gln Asn Glu Val Thr Leu Thr
   1625 1630 1635
   His Pro Ile Thr Lys Tyr Ile Met Ala Cys Met Ser Ala Asp Leu
   1640 1645 1650
   Glu Val Val Thr Ser Thr Trp Val Leu Val Gly Gly Val Leu Ala
   1655 1660 1665
   Ala Leu Ala Ala Tyr Cys Leu Thr Thr Gly Ser Val Val Ile Val
   1670 1675 1680
   Gly Arg Ile Ile Leu Ser Gly Arg Pro Ala Val Ile Pro Asp Arg
   1685 1690 1695
   Glu Val Leu Tyr Gln Glu Phe Asp Glu Met Glu Glu Cys Ala Ser
   1700 1705 1710
   His Leu Pro Tyr Ile Glu Gln Gly Met Gln Leu Ala Glu Gln Phe
   1715 1720 1725
   Lys Gln Lys Ala Leu Gly Leu Leu Gln Thr Ala Thr Lys Gln Ala
   1730 1735 1740
   Glu Ala Ala Ala Pro Val Val Glu Ser Lys Trp Arg Ala Leu Glu
   1745 1750 1755
   Thr Phe Trp Ala Lys His Met Trp Asn Phe Ile Ser Gly Ile Gln
   1760 1765 1770
   Tyr Leu Ala Gly Leu Ser Thr Leu Pro Gly Asn Pro Ala Ile Ala
   1775 1780 1785
   Ser Leu Met Ala Phe Thr Ala Ser Ile Thr Ser Pro Leu Ala Thr
   1790 1795 1800
   Gln Tyr Thr Leu Leu Phe Asn Ile Leu Gly Gly Trp Val Ala Ala
   1805 1810 1815
   Gln Leu Ala Pro Pro Ser Ala Ala Ser Ala Phe Val Gly Ala Gly
   1820 1825 1830
   Ile Ala Gly Ala Ala Val Gly Ser Ile Gly Leu Gly Lys Val Leu
   1835 1840 1845
   Val Asp Ile Leu Ala Gly Tyr Gly Ala Gly Val Ala Gly Ala Leu
   1850 1855 1860
   Val Ala Phe Lys Val Met Ser Gly Asp Met Pro Ser Thr Glu Asp
   1865 1870 1875
   Leu Val Asn Leu Leu Pro Ala Ile Leu Ser Pro Gly Ala Leu Val
   1880 1885 1890
   Val Gly Val Val Cys Ala Ala Ile Leu Arg Arg His Val Gly Pro
   1895 1900 1905
   Gly Glu Gly Ala Val Gln Trp Met Asn Arg Leu Ile Ala Phe Ala
   1910 1915 1920
   Ser Arg Gly Asn His Val Ser Pro Thr His Tyr Val Pro Glu Ser
   1925 1930 1935
   Asp Ala Ala Ala Arg Val Thr Gln Ile Leu Ser Asn Leu Thr Ile
   1940 1945 1950
   Thr Gln Leu Leu Lys Arg Leu His Gln Trp Ile Asn Glu Asp Cys
   1955 1960 1965
   Ser Thr Pro Cys Ser Gly Ser Trp Leu Arg Asp Val Trp Asp Trp
   1970 1975 1980
   Ile Cys Thr Val Leu Ala Asp Phe Lys Thr Trp Leu Gln Ser Lys
   1985 1990 1995
   Leu Leu Pro Arg Leu Pro Gly Val Pro Phe Phe Ser Cys Gln Arg
   2000 2005 2010
   Gly Tyr Lys Gly Val Trp Arg Gly Asp Gly Ile Met Tyr Thr Thr
   2015 2020 2025
   Cys Pro Cys Gly Ala Gln Ile Thr Gly His Val Lys Asn Gly Ser
   2030 2035 2040
   Met Arg Ile Val Gly Pro Arg Thr Cys Ser Asn Thr Trp His Gly
   2045 2050 2055
   Thr Phe Pro Ile Asn Ala Tyr Thr Thr Gly Pro Cys Thr Pro Ser
   2060 2065 2070
   Pro Ala Pro Asn Tyr Ser Arg Ala Leu Trp Arg Val Ala Ala Glu
   2075 2080 2085
   Glu Tyr Val Glu Val Thr Arg Val Gly Asp Phe His Tyr Val Thr
   2090 2095 2100
   Gly Met Thr Thr Asp Asn Val Lys Cys Pro Cys Gln Val Pro Ala
   2105 2110 2115
   Pro Glu Phe Phe Thr Glu Leu Asp Gly Val Arg Leu His Arg Tyr
   2120 2125 2130
   Ala Pro Ala Cys Lys Pro Leu Leu Arg Asp Glu Val Thr Phe Gln
   2135 2140 2145
   Val Gly Leu Asn Gln Tyr Thr Val Gly Ser Gln Leu Pro Cys Glu
   2150 2155 2160
   Pro Glu Pro Asp Val Thr Val Val Thr Ser Met Leu Thr Asp Pro
   2165 2170 2175
   Ser His Ile Thr Ala Glu Ala Ala Arg Arg Arg Leu Ala Arg Gly
   2180 2185 2190
   Ser Pro Pro Ser Leu Ala Gly Ser Ser Ala Ser Gln Leu Ser Ala
   2195 2200 2205
   Leu Ser Leu Lys Ala Thr Cys Thr Thr His His Gly Ala Pro Asp
   2210 2215 2220
   Thr Asp Leu Ile Glu Ala Asn Leu Leu Trp Arg Gln Glu Met Gly
   2225 2230 2235
   Gly Asn Ile Thr Arg Val Glu Ser Glu Asn Lys Ile Val Ile Leu
   2240 2245 2250
   Asp Ser Phe Glu Pro Leu Arg Ala Glu Glu Asp Glu Arg Glu Val
   2255 2260 2265
   Ser Ala Ala Ala Glu Ile Leu Arg Lys Thr Arg Lys Phe Pro Ala
   2270 2275 2280
   Ala Met Pro Val Trp Ala Arg Pro Asp Tyr Asn Pro Pro Leu Leu
   2285 2290 2295
   Glu Ser Trp Lys Asn Pro Asp Tyr Val Pro Pro Val Val His Gly
   2300 2305 2310
   Cys Pro Leu Pro Pro Thr Lys Ala Pro Pro Ile Pro Pro Pro Arg
   2315 2320 2325
   Arg Lys Arg Thr Val Val Leu Thr Glu Ser Thr Val Ser Ser Ala
   2330 2335 2340
   Leu Ala Glu Leu Ala Thr Lys Thr Phe Gly Gly Ser Gly Ser Ser
   2345 2350 2355
   Ala Val Asp Ser Gly Thr Ala Thr Gly Pro Pro Asp Gln Ala Ser
   2360 2365 2370
   Ala Glu Gly Asp Ala Gly Ser Asp Ala Glu Ser Tyr Ser Ser Met
   2375 2380 2385
   Pro Pro Leu Glu Gly Glu Pro Gly Asp Pro Asp Leu Ser Asp Gly
   2390 2395 2400
   Ser Trp Ser Thr Val Ser Glu Glu Ala Ser Glu Asp Val Val Cys
   2405 2410 2415
   Cys Ser Met Ser Tyr Thr Trp Thr Gly Ala Leu Ile Thr Pro Cys
   2420 2425 2430
   Ala Ala Glu Glu Ser Lys Leu Pro Ile Asn Ala Leu Ser Asn Pro
   2435 2440 2445
   Leu Leu Arg His His Asn Met Val Tyr Ser Thr Thr Ser Arg Ser
   2450 2455 2460
   Ala Ser Leu Arg Gln Lys Lys Val Thr Phe Asp Arg Met Gln Val
   2465 2470 2475
   Leu Asp Asp His Tyr Arg Asp Val Leu Lys Glu Met Lys Ala Lys
   2480 2485 2490
   Ala Ser Thr Val Lys Ala Lys Leu Leu Ser Val Glu Glu Ala Cys
   2495 2500 2505
   Lys Leu Thr Pro Pro His Ser Ala Lys Ser Lys Phe Gly Tyr Gly
   2510 2515 2520
   Ala Lys Asp Val Arg Ser Leu Ser Ser Arg Ala Val Asn His Ile
   2525 2530 2535
   Arg Ser Val Trp Lys Asp Leu Leu Glu Asp Thr Asp Thr Pro Ile
   2540 2545 2550
   Gln Thr Thr Ile Met Ala Lys Asn Glu Val Phe Cys Val Gln Pro
   2555 2560 2565
   Glu Lys Gly Gly Arg Lys Pro Ala Arg Leu Ile Val Phe Pro Asp
   2570 2575 2580
   Leu Gly Val Arg Val Cys Glu Lys Met Ala Leu Tyr Asp Val Val
   2585 2590 2595
   Ser Thr Leu Pro Gln Ala Val Met Gly Ser Ser Tyr Gly Phe Gln
   2600 2605 2610
   Tyr Ser Pro Lys Gln Arg Val Glu Phe Leu Val Asn Thr Trp Lys
   2615 2620 2625
   Ala Lys Lys Cys Pro Met Gly Phe Ser Tyr Asp Thr Arg Cys Phe
   2630 2635 2640
   Asp Ser Thr Val Thr Glu Asn Asp Ile Arg Val Glu Glu Ser Ile
   2645 2650 2655
   Tyr Gln Cys Cys Asp Leu Ala Pro Glu Ala Arg Gln Ala Ile Arg
   2660 2665 2670
   Ser Leu Thr Glu Arg Leu Tyr Ile Gly Gly Pro Met Thr Asn Ser
   2675 2680 2685
   Lys Gly Gln Asn Cys Gly Tyr Arg Arg Cys Arg Ala Ser Gly Val
   2690 2695 2700
   Leu Thr Thr Ser Cys Gly Asn Thr Leu Thr Cys Tyr Leu Lys Ala
   2705 2710 2715
   Ala Ala Ala Cys Arg Ala Ala Lys Leu Gln Asp Cys Thr Met Leu
   2720 2725 2730
   Val Cys Gly Asp Asp Leu Val Val Ile Cys Asp Ser Ala Gly Thr
   2735 2740 2745
   Gln Glu Asp Ala Ala Ser Leu Arg Val Phe Thr Glu Ala Met Thr
   2750 2755 2760
   Arg Tyr Ser Ala Pro Pro Gly Asp Pro Pro Gln Pro Glu Tyr Asp
   2765 2770 2775
   Leu Glu Leu Ile Thr Ser Cys Ser Ser Asn Val Ser Val Ala His
   2780 2785 2790
   Asp Ala Ser Gly Lys Arg Val Tyr Tyr Leu Thr Arg Asp Pro Thr
   2795 2800 2805
   Thr Pro Leu Ala Arg Ala Ala Trp Glu Thr Ala Arg His Thr Pro
   2810 2815 2820
   Val Asn Ser Trp Leu Gly Asn Ile Ile Met Tyr Ala Pro Thr Leu
   2825 2830 2835
   Trp Ala Arg Met Ile Leu Met Thr His Phe Phe Ser Ile Leu Leu
   2840 2845 2850
   Ala Gln Glu Gln Leu Glu Lys Ala Leu Asp Cys Gln Ile Tyr Gly
   2855 2860 2865
   Ala Thr Tyr Ser Ile Glu Pro Leu Asp Leu Pro Gln Ile Ile Gln
   2870 2875 2880
   Arg Leu His Gly Leu Ser Ala Phe Ser Leu His Ser Tyr Ser Pro
   2885 2890 2895
   Gly Glu Ile Asn Arg Val Ala Ser Cys Leu Arg Lys Leu Gly Val
   2900 2905 2910
   Pro Pro Leu Arg Val Trp Arg His Arg Ala Arg Ser Val Arg Ala
   2915 2920 2925
   Lys Leu Leu Ser Gln Gly Gly Arg Ala Ala Thr Cys Gly Lys Tyr
   2930 2935 2940
   Leu Phe Asn Trp Ala Val Lys Thr Lys Leu Lys Leu Thr Pro Ile
   2945 2950 2955
   Pro Glu Ala Ser Gln Leu Asp Leu Ser Gly Trp Phe Val Ala Gly
   2960 2965 2970
   Tyr Ser Gly Gly Asp Ile Tyr His Ser Leu Ser Arg Ala Arg Pro
   2975 2980 2985
   Arg Trp Phe Met Trp Cys Leu Leu Leu Leu Ser Val Gly Val Gly
   2990 2995 3000
   Ile Tyr Leu Leu Pro Asn Arg
   3005 3010

## Description

### TECHNICAL FIELD

The present invention relates to a method for inducing cytotoxic T-cells, a cytotoxic T-cell inducer, and a pharmaceutical composition and a vaccine employing same.

### BACKGROUND ART

When infection with a virus such as a hepatitis C virus (HCV) occurs, a virus elimination reaction due to natural immunity proceeds, a specific immune response is subsequently induced, and a virus elimination reaction proceeds.

In the specific immune response, virus in a body fluid is eliminated by a neutralizing antibody, and intracellular virus is eliminated by cytotoxic T-cells (CTL). That is, the CTL specifically recognizes a virus antigen peptide (CTL epitope) consisting of 8 to 11 amino acids presented in an HLA class I molecule on the surface of an infected cell, and eliminates the virus by damaging the infected cell. Identifying such a virus-specific CTL epitope is therefore important when investigating the specific immune response.

Identification of this CTL epitope, and methods for ascertaining immunity-inducing capability, are described in Non-patent Publications 1 to 4.
[Non-patent Publication 1] Battergay, M., J. Fikes, et al. (1995). 'Patients with Chronic Hepatitis C Have Circulating Cytotoxic T Cells Which Recognize Hepatitis C Virus-Encoded Peptides Binding to HLA-A2.1 Molecules. J Virol 69: pp. 2462-2470
[Non-patent Publication 2] Cerny, A., J. McHutchinson, et al. (1995). 'Cytotoxic T Lymphocyte Response to Hepatitis C Virus-derived Peptides Containing the HLA A2.1 Binding Motif.' J Clin Invest 95: pp. 521-530
[Non-patent Publication 3] Kurokohchi, K., K. Arima, et al. (2001). 'A novel cytotoxic T-cell epitope presented by HLA-A24 molecule in hepatitis C virus infection.' J Hepatology 34: pp. 930-935
[Non-patent Publication 4] Nakamoto, Y., S. Kaneko, et al. (2003). 'Analysis of the CD8-Positive T Cell Response in Japanese Patients With Chronic Hepatitis C Using HLA-A*2402 Peptide Tetramers.' J Med Virol 70: pp. 51-61

WO2005/118626 describes peptides derived from the Hepatitis C Virus (HCV) and nucleic acids which encodes them. The peptides are those which elicit a cytotoxic and/or helper T lymphocyte response in a host. WO2005/118626 also describes vaccines for prevention and treatment of HCV injection and diagnostic methods for detection of HCV exposure in patients.

### DISCLOSURE OF THE INVENTION

### PROBLEMS TO BE SOLVED BY THE INVENTION

The above publications cite HLA-binding peptides with a 2 to 5 sequence peptides that induce cytotoxic T-cells. On the other hand, it is known that hepatitis C virus mutates frequently once it has entered the human body. Therefore, it could well happen that immunotherapy using peptides described in the above publications may not be effective to such mutated viruses. In order to treat and prevent a disease caused by hepatitis C virus, it is desired that more peptides are identified in order to increase the option of choosing alternative peptides which have the capability of inducing cytotoxic T-cells.

The present invention has been accomplished in the light of the above-mentioned circumstances, and it is an exemplary object thereof to provide a method for inducing cytotoxic T-cells, a cytotoxic T-cell inducer, and a pharmaceutical composition and a vaccine employing same, that enable a disease that is caused by hepatitis C virus to be treated and prevented effectively.

### MEANS FOR SOLVING THE PROBLEMS

In one exemplary aspect of the invention, there is provided a method for inducing cytotoxic T-cells *in vitro,* the method comprising: generating a target cell for cytotoxic T-cells on which surface HLA molecules are bound by an amino acid sequence consisting of SEQ ID NO: 1.

Furthermore, in another exemplary aspect of the invention, there is provided a cytotoxic T-cell inducer for use in a method of inducing cytotoxic T-cells, the cytotoxic T-cell inducer comprising a peptide, the peptide consisting of an amino acid sequence consisting of SEQ ID NO: 1, wherein the method of inducing cytotoxic T-cells comprises a step of binding the peptide to an HLA molecule on the surface of a cell that is a target of a cytotoxic T-cell.

Moreover, in another exemplary aspect of the invention, there is provided a pharmaceutical composition for use in a method of treating a disease that is caused by hepatitis C virus, the composition comprising a peptide, the peptide consisting of an amino acid sequence consisting of SEQ ID NO: 1, wherein the method of treating comprises a step of binding the peptide to an HLA molecule on the surface of a cell infected with hepatitis C virus.

Furthermore, in another exemplary aspect of the invention, there is provided a vaccine for use in a method of preventing or treating a disease that is caused by hepatitis C virus, the vaccine comprising a peptide, the peptide consisting of an amino acid sequence consisting of SEQ ID NO: 1, wherein the method comprises a step of binding the peptide to an HLA molecule on the surface of a cell infected with hepatitis C virus.

### EFFECTS OF THE INVENTION

In an exemplary advantage according to the invention, since cytotoxic T-cells can be induced effectively, a pharmaceutical composition and a vaccine that are useful in particular for the treatment or prevention of a disease that is caused by hepatitis C virus can be obtained.

### BRIEF DESCRIPTION OF THE DRAWINGS

The above-mentioned object, other objects, features, and advantages will become more apparent from exemplary embodiments explained below by reference to the attached drawings.
[FIG. 1] A schematic drawing for explaining an active learning experiment design used in an example.
[FIG. 2] A graph showing the results of an investigation of cytotoxic T-cell-inducing capability in an example.
[FIG. 3] A graph showing the results of an investigation of cytotoxic T-cell-inducing capability in an example.
[FIG. 4] A graph showing the results of an investigation of damage to hepatitis C virus-expressing cells in an example.

### EXEMPLARY EMBODIMENT

Exemplary embodiments for carrying out the present invention are explained below using the drawings.

### A first exemplary embodiment

The method for inducing cytotoxic T-cells related to the present exemplary embodiment includes binding, to an HLA molecule on the surface of a cell that is a target of
a cytotoxic T-cell, a peptide containing the amino acid sequence having the SEQ ID NO: 1 and consisting of not less than 8
and not more than 11 amino acid residues, or a peptide derived from a precursor thereof (hereinafter, called an 'HLA-binding peptide').

The amino acid sequences shown in SEQ ID NO: 1 is a sequence consisting of 9 amino acid residues contained in a certain genome protein of a hepatitis C virus (HCV) shown in SEQ ID NOS: 15 and 16.

Furthermore, among amino acid sequences for which the binding to an HLA molecule, predicted by a hypothesis obtained using an active learning experiment method (Japanese Patent Publication No. JP2001-129191, A,
is 3 or greater in terms of a -log Kd value,
these amino acid sequences have actually been confirmed by an HLA molecule binding experiment to exhibit HLA binding and have been shown to be capable of inducing cytotoxic T-cells.

When selecting a candidate, an amino acid sequence for which the binding to an HLA molecule in terms of a - log Kd value is 3 or greater is selected from the viewpoint that in the field of biochemistry it is known that a binding ability, in terms of a -log Kd value, of about 3 can be treated as the threshold level for whether or not a peptide actually binds to an MHC, which includes an HLA.

That is, the peptide having the amino acid sequence shown in SEQ ID NO: 1 corresponds to an epitope of an antigen peptide presented in an HLA molecule on the surface of a cell infected with hepatitis C virus.

It has been confirmed that the peptide having the amino acid sequences shown in SEQ ID NO: 1 binds to a product (HLA-A24 molecule) of the HLA-A*2402 gene, which is one of the alleles of gene A, and about 50% of Japanese people have an HLA-A24 molecule of a subclass of the gene A.

The sequences of SEQ ID NOS: 1 to 13 and the sequence of SEQ ID NO: 14 for comparison therewith are shown in Table 1 below.

**[Table 1]**

| SEQ ID No | SEQ | Predicted Score | SEQ Name | Binding Experiment Data |
|---|---|---|---|---|
| 1 | LLPRRGPRL | 5.30 | 36D90D89 | 7.71 |
| 2 | WHYPCTVNF | 5.66 | 616D90D89 | 6.39 |
| 3 | LLSTTEWQI | 5.35 | 666D90D89 | 8.34 |
| 4 | ILPCSFTTL | 6.75 | 674D90 | 7.66 |
| 5 | ALYGVWPLL | 5.80 | 789D90 | 6.98 |
| 6 | YYKVFLARL | 5.49 | 834D90 | 7.12 |
| 7 | VFSDMETKL | 5.54 | 975D90 | 7.31 |
| 8 | AYSQQTRGL | 5.76 | 1031D90D89 | 6.54 |
| 9 | ITYSTYCKF | 5.27 | 1291D90 | 6.98 |
| 10 | RYAPACKPL | 5.47 | 2132D89 | 6.76 |
| 11 | SMLTDPSHI | 5.55 | 2173D90D89 | 6.94 |
| 12 | SYTWTGALI | 5.51 | 2422D90D89 | 7.13 |
| 13 | ILMTHFFSI | 5.57 | 2843D90D89 | 7.90 |
| 14 | FWAKHMWNF | 5.93 | 1760D90D89 | 8.10 |

Among the sequences of SEQ ID NOS: 1 to 14, those having a sequence name with 'D90' denote sequences consisting of 9 amino acid residues contained in a certain genome protein (SEQ ID NO: 15) of HCV D90208 strain, which will be described later. Furthermore, those having a sequence name with 'D89' denote sequences consisting of 9 amino acid residues contained in a certain genome protein (SEQ ID NO: 16) of HCV D89815 strain, which will be described later. Moreover, those with 'D90D89' denote sequences contained in common in both of the above. Furthermore, in the table, predicted scores and binding experiment data with respect to binding to the HLA-A24 molecule of each sequence are shown in terms of -log Kd values. As can be seen, a correlation exists between the predicted score and the binding experiment data.

Since there is no conventional technique for discovering an HLA-binding peptide by utilizing such an experimental design method, there are only a very small number of HLA-binding peptides that have been experimentally confirmed to have HLA-binding properties. Because of this, even when a peptide consisting of 9 amino acid residues is randomly synthesized by a conventional method and subjected to an experiment to find out if it binds to an HLA molecule, there is a probability of only about 1 in 100 of finding one that has a binding, in terms of a -log Kd value, exceeding 6.

Furthermore, among candidates predicted by the active learning experiment method as described later, a peptide having the amino acid sequence of SEQ ID NO: 14 showed high binding in an HLA molecule binding experiment but did not show a capability of inducing cytotoxic T-cells. On the other hand, the peptides having the amino acid sequences of SEQ ID NOS: 1 to 13 showed a capability of inducing cytotoxic T-cells.

As described above, in order to induce cytotoxic T-cells, not only predicting the sequence of an HLA-binding peptide by the active learning experiment method but also selecting a specific amino acid sequence among those predicted candidates are important factors.

In the present exemplary embodiment, a peptide having an amino acid sequence of SEQ ID NO: 1 is introduced into a system containing cells that are the target of cytotoxic T-cells (CTL), binds to an HLA class I molecule on the surface of the cell, and is presented to the cytotoxic T-cell as an antigen peptide. The cytotoxic T-cell specifically recognizes this and is induced. Furthermore, the induced cytotoxic T-cell damages the cell that presents the antigen peptide. In this way, the peptide having an amino acid sequence of SEQ ID NO: 1
functions as a virus antigen (CTL epitope) for inducing the cytotoxic T-cell.

The 'inducing' referred to here means generating an activity or an action from a material or a state in which there is almost no activity or action. In particular, 'inducing a cytotoxic T-cell' means differentiating the cytotoxic T-cells that specifically recognize a certain antigen into effector cells having the capability of killing target cells, and/or proliferating the cytotoxic T-cells, in vitro or in vivo.

From another viewpoint, by containing at least one of a peptide and a precursor thereof, the peptide containing the amino acid sequence of SEQ ID NO: 1 consisting of not less than 8 and not more than 11 amino acid residues, and binding to an HLA molecule on the surface of a cell that is a target of a cytotoxic T-cell, a cytotoxic T-cell inducer can be obtained.

The 'cytotoxic T-cell inducer' means a drug that exhibits an action of changing a state in which CD8 positive T-cells specifically recognizing a certain antigen are not present, or are present only at a very low proportion, into a state in which cytotoxic T-cells recognizing this antigen are present at a very high proportion, and an action of enhancing the capability of individual cytotoxic T-cells of killing a target.

Furthermore, among HLA-binding peptides containing an amino acid sequence that is predicted by the active learning experiment method and confirmed by the binding experiment to actually bind, since only some thereof induced cytotoxic T-cells, it is surmised that, although it remains important for a peptide to bind to an HLA molecule on the surface of the cell in order to induce cytotoxic T-cells, a difference in T-cell repertoire between individuals and the like or another factor is acting.

HLA-binding peptides used in the present exemplary embodiment include those formed by modifying a sequence or a site that does not affect the binding to an HLA molecule in a peptide having a sequence of SEQ ID NO: 1 by
chemical modification or substitution with a different stereoisomer of an amino acid used in the HCV genome protein. Furthermore, such an HLA peptide may be a peptide consisting of amino acid residues alone as described above, but it is not particularly limited thereto. For example, it may be an HLA-binding peptide precursor that is optionally modified with a sugar chain or a fatty acid group and the like as long as the effects of the present invention are not impaired. Such a precursor is subjected to a change involving digestion by a proteolytic enzyme and the like in a living mammalian body such as in a human digestive organ to become an HLA-binding peptide, thus exhibiting similar effects to those shown by the above-mentioned HLA-binding peptide.

In the present exemplary embodiment, since cytotoxic T-cells can be induced by a peptide that binds to the HLA-A24 molecule, which is often seen in Asian people, such as Japanese people, this can be utilized in the development of a therapeutic drug, a prophylactic drug, and the like that is particularly effective for Asian people, such as Japanese people. The proportion of people having the HLA-A24 molecule is as low as 10 or so percent in Europe and America, but it is still one of the main HLA genotypes, and it is important as a target molecule for an immunotherapeutic drug.

Moreover, the HLA-binding peptides used in the present exemplary embodiment may be produced using a method known to a person skilled in the art. For example, they may be artificially synthesized by a solid-phase method or a liquid-phase method. Alternatively, these HLA-binding peptides may be produced by expressing them from a DNA fragment or a recombinant vector coding for these HLA-binding peptides. These HLA-binding peptides thus obtained can be identified by a method known to a person skilled in the art. For example, identification is possible by use of Edman degradation, mass spectrometry, and the like.

### A second exemplary embodiment

The pharmaceutical composition related to the present exemplary embodiment contains the cytotoxic T-cell inducer explained in Embodiment 1. That is, this pharmaceutical composition is a pharmaceutical composition for the treatment of a disease that is caused by hepatitis C virus, the composition containing at least one of a peptide and a precursor thereof, the peptide containing one or more types of amino acid sequence selected from the group consisting of SEQ ID NO: 1, consisting of not less than 8 and not more than 11 amino acid residues, and binding to an HLA molecule on the surface of a cell expressing hepatitis C virus (hereinafter, called an 'expressing cell').

The peptide having an amino acid sequence of SEQ ID NOS: , as explained in Embodiment 1, has HLA-binding properties and can induce cytotoxic T-cells.

In the explanation of Embodiment 1, cytotoxic T-cells can be induced by using the expressing cells as the target cells for the cytotoxic T-cells, and the expressing cells are damaged by these induced cytotoxic T-cells.

That is, by administering the pharmaceutical composition of the present exemplary embodiment to a patient with a disease that is caused by hepatitis C virus, the peptide contained in the composition binds to an HLA class I molecule on the surface of cells within the patient's body, and is presented as an antigen peptide to cytotoxic T-cells. The cytotoxic T-cells specifically recognize this and are activated. In this way, the cytotoxic T-cells are induced. Furthermore, the induced cytotoxic T-cells damage the infected cells presenting the antigen peptide, and this function enables a contribution to be made to the treatment of hepatitis C.

The HLA-binding peptide contained in the present exemplary embodiment may be a peptide consisting of amino acid residues alone as described above, but it is not particularly limited thereto. For example, it may be an HLA-binding peptide precursor that is optionally modified with a sugar chain or a fatty acid group and the like as long as the effects of the present invention are not impaired. Such a precursor is subjected to a change involving digestion by a proteolytic enzyme and the like in a living mammalian body such as in a human digestive organ to become an HLA-binding peptide, thus exhibiting similar effects to those shown by the above-mentioned HLA-binding peptide. Furthermore, such HLA-binding peptides may be produced by a method known to a person skilled in the art. For example, they may be artificially synthesized by a solid phase method or a liquid phase method.

The pharmaceutical composition of the present exemplary embodiment may be administered to a patient by dissolving it in a water-soluble solvent and made into a preparation in the form of a pharmaceutically acceptable salt.

Examples of the form of such a pharmaceutically acceptable salt include water-soluble salts that are physiologically acceptable, such as sodium, potassium, magnesium, and calcium salts that are buffered at a physiological pH. Other than the water-soluble solvent, a water-insoluble solvent may be used, and examples of such a water-insoluble solvent include alcohols such as ethanol and propylene glycol.

A preparation containing the pharmaceutical composition of the present exemplary embodiment may contain agents for various purposes, and examples of such agents include a preservative and a buffer agent.

Examples of the preservative include sodium bisulfite, sodium bisulfate, sodium thiosulfate benzalkonium chloride, chlorobutanol, thimerosal, phenylmercuric acetate, phenylmercuric nitrate, methylparaben, polyvinyl alcohol, phenylethyl alcohol, ammonia, dithiothreitol, and β-mercaptoethanol. Examples of the buffer agent include sodium carbonate, sodium borate, sodium phosphate, sodium acetate, and sodium bicarbonate. These agents can be present in an amount that enables the pH of the system to be maintained at between 2 and 9, and preferably at between 4 and 8.

### A third exemplary embodiment

A vaccine related to the present exemplary embodiment contains the pharmaceutical composition explained in the second exemplary embodiment. That is, this vaccine is a vaccine used for the prevention or treatment of a disease that is caused by hepatitis C virus, the vaccine containing at least one of a peptide and a precursor thereof, the peptide containing the amino acid sequence SEQ ID NO. 1, consisting of not less than 8 and not more
than 11 amino acid residues, and binding to an HLA molecule on the surface of a cell expressing hepatitis C virus (hereinafter, called an 'expressing cell').

The peptide having the amino acid sequence of SEQ ID NO: 1
contained in the pharmaceutical composition, as explained in the first exemplary embodiment, has HLA-binding properties and can induce cytotoxic T-cells.

As in the second exemplary embodiment, in the explanation of the first exemplary embodiment, cytotoxic T-cells can be induced by using the HCV expressing cells as the target cells for the cytotoxic T-cells, and the HCV expressing cells are damaged by these induced cytotoxic T-cells.

That is, by administering the vaccine of the present exemplary embodiment to a patient with hepatitis C virus, the peptide contained in the composition binds to an HLA-A24 molecule on the surface of tissue cells of the patient, including dendritic cells always present at an injection site. When cytotoxic T-cells specific to the peptide recognize it, they are activated, proliferate, and circulate via general circulation. When cytotoxic T-cells specific to the peptide enter the expressing liver tissue, they recognize the same virus-derived peptide naturally binding to an HLA-A24 molecule present on the surface of hepatitis C virus-expressing liver cells, and kill the expressing cells, thus cutting off the virus source. This function enables a contribution to be made to the treatment of a disease that is caused by hepatitis C virus.

Alternatively, by administering the vaccine of the present exemplary embodiment to a healthy human body, cytotoxic T-cells are induced, the induced cytotoxic T-cells build up within the body, and when hepatitis C virus enters, cells expressing this hepatitis C virus can be killed. This function enables a contribution to be made to the prevention of a disease that is caused by hepatitis C virus.

The HLA-binding peptide contained in the present exemplary embodiment may be a peptide consisting of amino acid residues alone as described above, but it is not particularly limited thereto. For example, it may be an HLA-binding peptide precursor that is optionally modified with a sugar chain or a fatty acid group and the like as long as the effects of the present invention are not impaired. Such a precursor is subjected to a change involving digestion by a proteolytic enzyme and the like in a living mammalian body such as in a human digestive organ to become an HLA-binding peptide, thus exhibiting similar effects to those shown by the above-mentioned HLA-binding peptide. Furthermore, such HLA-binding peptides may be produced by a method known to a person skilled in the art. For example, they may be artificially synthesized by a solid phase method or a liquid phase method.

Furthermore, the vaccine of the present exemplary embodiment may be used in the form of an inactive component-containing vaccine that contains a component, other than components of the pharmaceutical composition, that has no activity itself but has an effect in yet further enhancing the effect of the pharmaceutical composition as a vaccine. Examples of the inactive component include an adjuvant and a toxoid.

The pharmaceutical composition of the second exemplary embodiment and the vaccine of the third exemplary embodiment are internally administered by injection or infusion via subcutaneous, intravenous, or intramuscular administration, and the like, by percutaneous administration, or by inhalation via the mucosa of the nose, throat, and the like.

The amount thereof per administration may be set between an amount that can significantly induce cytotoxic T-cells and an amount that does not damage a significant number of uninfected cells.

### (Example 1)

The present invention is further explained below by reference to Examples wherein the peptide is SEQ ID NO: 1, but the present invention is not limited thereto.

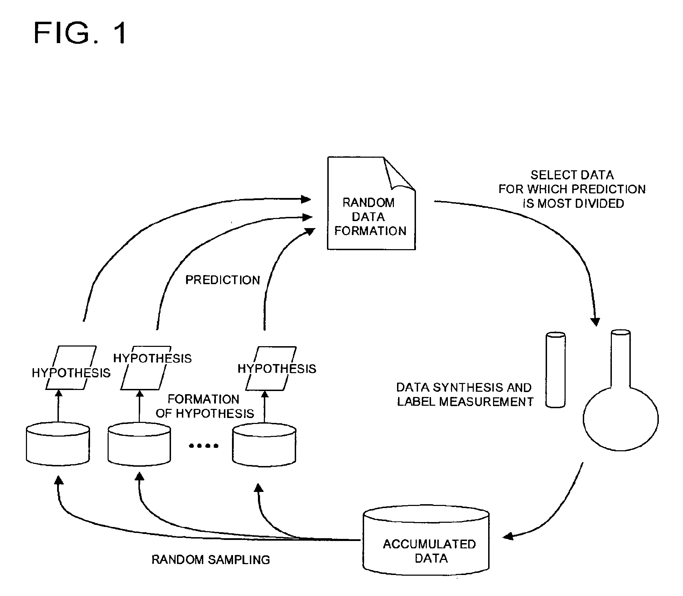

Specifically, procedures of prediction, experiment, and evaluation in the present Examples were carried out based on an active learning experiment design described in WO2006/004182, and in general the following steps were repeated, thus creating rules. A schematic drawing for the active learning experiment design employed here is shown in FIG. 1.
(1) A trial of a lower-order learning algorithm, which will be described later, was carried out once. That is, a plurality of hypotheses were generated by random sampling from accumulated data and, with regard to randomly expressed candidate query points (peptides), a point that showed the largest distribution of predicted values was selected as a query point to be subjected to an experiment.
(2) The peptide at the selected query point was prepared by a synthesis and purification method, which will be described later, and the actual binding ability was measured by an experiment, which will be described later, and added to accumulated data.

According to such an active learning method, the number of repetitions of the binding experiment for peptides consisting of 9 amino acid residues, which would otherwise have to be carried out for the 500,000,000,000 (= 20⁹) or more combinations of all the candidates for HLA-binding peptides, could be reduced.

Amino acid sequences shown in SEQ ID NOS: 1 to 14 were extracted by the rules explained above.

### <Synthesis and purification of peptide>

A peptide having an amino acid sequence of SEQ ID NOS: 1 to 14 was manually synthesized by the Merrifield solid-phase method using Fmoc amino acids. After deprotection, reverse phase HPLC purification was carried out using a C18 column to give a purity of 95% or higher. Identification of the peptide and confirmation of its purity were carried out using a MALDI-TOF mass spectrometer (Voyager DE RP, PerSeptive). Quantitative analysis of the peptide was carried out by a Micro BCA assay (Pierce Corp.) using BSA as a standard protein.

### <Experiment of binding peptide to HLA-A24 molecule>

The ability of a peptide having an amino acid sequence of SEQ ID NOS: 1 to 14 to bind to an HLA-A24 molecule, which is a product of the HLA-A*2402 gene, was measured using C1R-A24 cells expressing the HLA-A24 molecule (cells produced by associate professor Masafumi Takiguchi, Kumamoto University being supplied with permission by Assistant Professor Masaki Yasukawa, Ehime University).

C1R-A24 cells were first exposed to acidic conditions at a pH of 3.3 for 30 seconds, thus dissociating and removing a light chain β2m, which is associated with HLA class I molecules in common, and an endogenous peptide originally bound to the HLA-A*2402 molecule. After neutralization, purified β2m was added to C1R-A24 cells, the obtained product was added to serial dilutions of a peptide, and incubated on ice for 4 hours. Staining was carried out using fluorescently labeled monoclonal antibody 17A12, which recognizes association (MHC-pep) of the three members, that is, the HLA-A*2402 molecule, the peptide, and β2m, which had reassociated during the incubation.

Subsequently, the MHC-pep count per C1R-A24 cell (proportional to the strength of fluorescence of the above-mentioned fluorescent antibody) was quantitatively measured using a FACScan fluorescence-activated flow cytometer (Becton Dickinson Biosciences). A binding dissociation constant Kd value between the HLA-A24 molecule and the peptide was calculated from the average strength of fluorescence per cell by a published method (Udaka et al., Immunogenetics, 51, 816-828, 2000).

### <Evaluation results from binding experiment>

The prediction results and the experimental results shown in Table 1 above were obtained.

The sequences of SEQ ID NOS: 1 to 3, the sequence of SEQ ID NO: 8, and the sequences of SEQ ID NOS: 11 to 14 in Table 1 are sequences consisting of 9 amino acid residues contained in common in both the full-length sequence (SEQ ID NO: 15) of a certain genome protein of HCV D90208 strain registered in GENBANK and the full-length sequence (SEQ ID NO: 16) of a certain genome protein of HCV D89815 strain similarly registered in GENBANK.

Furthermore, the sequences of SEQ ID NOS: 4 to 7 and the sequence of SEQ ID NO: 9 are sequences consisting of 9 amino acid residues contained only in the full-length sequence (SEQ ID NO: 15) of the certain genome protein of the HCV D90208 strain. Moreover, SEQ ID NO: 10 is a sequence consisting of 9 amino acid residues contained only in the full-length sequence (SEQ ID NO: 16) of the certain genome protein of the HCV D89815 strain.

The full-length amino acid sequence of the certain genome protein of the HCV D90208 strain is shown in SEQ ID NO: 15

Furthermore, the full-length amino acid sequence of the certain genome protein of the HCV D89815 strain is shown in SEQ ID NO: 16

### <Cytotoxic T-cell induction experiment>

The following experiment was carried out using a human blood sample.

### (1) Activation of cytotoxic T-cells

Blood was sampled from a hepatitis C patient or a healthy individual from whom informed consent had been obtained in advance.

To blood cells (Peripheral blood mononuclear cells: PBMCs) sown on 96 well plates peptides of SEQ ID NOS: 1 to 14 were added weekly for 5 weeks so as to give final concentrations of 1 μM, and cytotoxic T-cells were activated in media to which was added 10% FCS and 5 ng/mL of IL-2, thus giving T-cell samples.

### (2) Treatment of ⁵¹Cr-labeled cells

Cells were labeled with ⁵¹Cr by adding the sodium salt of ⁵¹Cr (sodium chromate) to C1R-A24 cells and T2-A24 cells (human B/T cultured cell strain having no TAP peptide transporter and expressing the HLA-A*2402 gene: supplied by Dr. Tsuboi, Faculty of Medicine, Osaka University) at 37°C or 26°C for 1 hour. Peptides of SEQ ID NOS: 1 to 14 were added thereto so as to make final concentrations of 1 μM, thus forming target cells having each peptide binding to the surface of the cells.

### (3) Damaging of labeled cells

After the cytotoxic T-cells and the target cells prepared above were mixed at an E/T ratio (effector to target) of 10 to 20 and allowed to stand at 37° C for 3.5 hours, the radioactivity of ⁵¹Cr released in the culture supernatant was measured. The influence of nonspecific activity that does not depend on the action of cytotoxic T-cells was subtracted, and the cytolytic activity against the labeled cells was calculated.

### <Results of induction experiment>

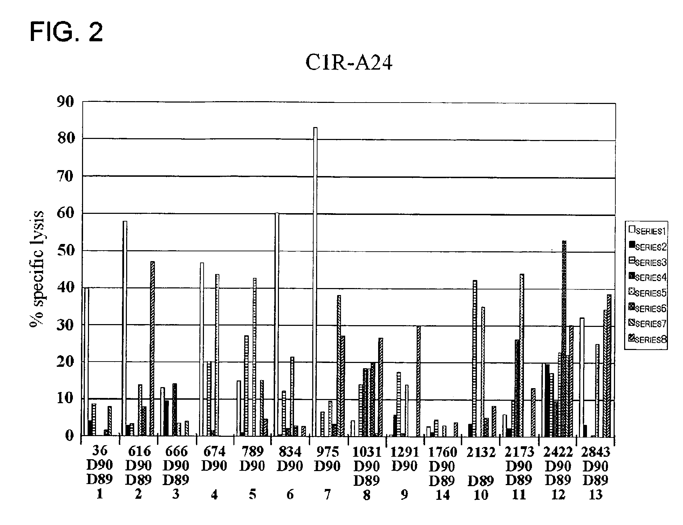

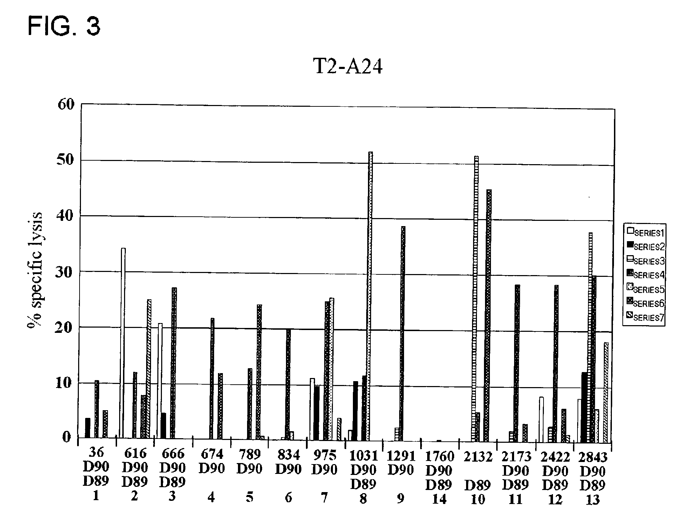

FIG. 2 and FIG. 3 show the results of cytotoxic T-cell induction experiments of peptides with each amino acid sequence in T-cell samples cultured in separate media. In the figures, data of blood samples derived from 8 people (or 7 people) are shown in 'series 1' to 'series 8 (or series 7)'.

With regard to the sequences of SEQ ID NOS: 1 to 13, blood from any one of the 8 people showed a capability of inducing cytotoxic T-cells. In contrast, with regard to the sequence of SEQ ID NO: 14, none of the blood samples showed a capability of inducing cytotoxic T-cells. This trend was seen in both FIG. 2 and FIG. 3 in common.

It is surmised that the reason why the same capability for inducing cytotoxic T-cells was not shown for blood from all of the 8 people by the amino acid sequences of SEQ ID NOS: 1 to 13 in both FIG. 2 and FIG. 3 is that, although there is no difference in binding to an HLA molecule between individuals, since the T-cell repertoire of an individual differs depending on individual genetic background or past infection history, there is a possibility of individual differences in peptide reactivity appearing.

Therefore, FIG. 2 and FIG. 3 suggest that, from the viewpoint of responding to a large number of patients, it is preferable to use a plurality of types of amino acid at the same time.

### [Example 2]

### <Damaging activity targeted at HCV-expressing cells>

### (1) Preparation of HCV genome-expressing cells

RzM6 cells were formed by transfecting HepG2, which is a human liver cell strain, with an HCV gene that had been modified so as to be able to conditionally induce expression of an HCV1b full genome (AY045702) in the presence of Cre (Activation of the CK1-CDK-Rb-E2F pathway in full genome hepatitis C virus-expressing cells. Tsukiyama-Kohara et al., J. Biol. Chem. 279, 14531-14541, 2004, RzM6 being supplied by Dr. Michinori Obara, Tokyo Metropolitan Institute of Medical Science).

In the HCV expression method, since the same RzM6 cells are doubly transfected with the Cre gene inserted in a conditional expression cassette arranged downstream of a Tamoxifen-inducible promoter, by adding tamoxifen, expression of Cre is induced, and as a result expression of the HCV gene can be induced. The RzM6 cells for which expression of the HCV gene was induced were labeled with ⁵¹Cr by the same method as for the C1R-A24 cells, thus serving as target cells.

### (2) Damage to HCV genome-expressing cells

The cytotoxic T-cells prepared above and RzM6 target cells or HepG2 cells with no HCV introduced as a control were mixed at an E/T ratio of 10 and allowed to stand at 37°C for 3 hours, and the radioactivity of ⁵¹Cr released into the culture supernatant was measured. The influence of nonspecific activity that does not depend on the action of cytotoxic T-cells was subtracted, and the cytolytic activity against target cells was calculated.

### <Results of cytolysis assay against HCV genome-expressing cells>

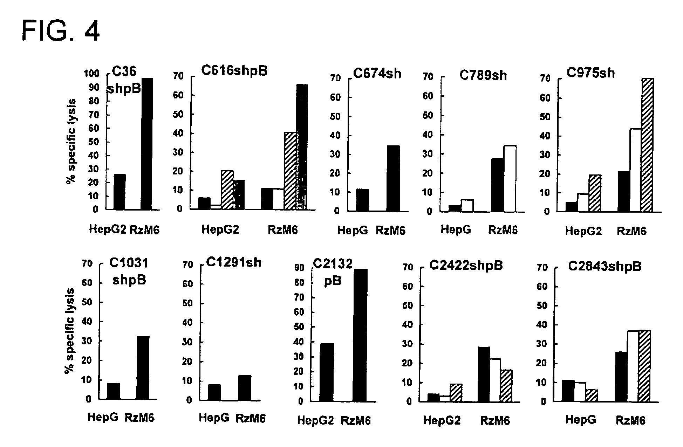

FIG. 4 shows the results of a cytolysis assay against HCV genome-expressing RzM6 cells with a cell line which was grown from peripheral blood T-cells were taken from different people and stimulated by the peptides of each of the amino acid sequences so as to increase reactive T-cells. Each bar of the graph shows the cytolytic activity of individual cell line grown as above against RzM6 cells.

With regard to the sequences of SEQ ID NOS: 1, 2, 4, 5, 7, 8, 9, 10, 12, and 13, a cytolytic activity specific to HCV-expressing cells by the cytotoxic T-cells was exhibited for the blood from any one of the patients.

### [Example 3]

### <Animal model used>

HCV has the property that it does not replicate in anything other than humans or chimpanzees. However, when chimpanzees are used as an animal model, although some develop chronic hepatitis, there is a tendency for it to be cured as acute hepatitis, and the pathophysiology is not the same as for human beings. Moreover, a very large expenditure is required, it takes at least one year for the onset of chronic hepatitis, it is ethically difficult to sacrifice the chimpanzee after use, and it is in practice impossible to carry out an infection experiment in a normal research organization. As a substitute animal model, a system in which a small animal such as a mouse is infected using an expression vector that has been modified so as to express the HCV gene is utilized.

### <Experimental materials>

### (1) HCV transgenic mouse (CN2-29)

A transgenic mouse that conditionally expresses only a portion (nt: 294-3435) corresponding to the front 1/3 of the HCV genome, produced by Dr. Michinori Obara, Tokyo Metropolitan Institute of Medical Science, is used (reference: Efficient conditional transgene expression in Hepatitis C virus cDNA transgenic mice mediated by the Cre/loxP system. Wakita et al., J. Biol. Chem. 273, 9001-9006, 1998.).

### (2) Mouse in which good T-cell induction occurs (CBF1)

The CN2-29 mouse has a BALB/c mouse (H-2^{d}) background, and K^{d}, D^{d}, L^{d}-binding peptides are target epitopes. Mice were immunized using these binding peptides, and induction of T-cells was attempted, but only weak cytotoxicity was induced. Since one with good T-cell induction was found for the D^{b}-binding peptide, if an F1 mouse (CBF1) produced by crossbreeding the CN2-29 mouse with a C57BL/6 (abbreviated to B6, H-2^{b}) mouse is used as a host, in vivo cytotoxicity can be examined.

### (3) EGFP transgenic mouse

A transgenic mouse having a B6 mouse genetic background and expressing EGFP (jellyfish-derived fluorescent gene) ubiquitously in the whole body.

### (4) Cre expression vector (BCre)

An expression vector formed by incorporating the Cre gene into a bovine papilloma virus-based expression vector called BCMGSneo (reference: Establishment of mouse cell lines which constitutively secrete large quantities of interleukin 2, 3, 4 or 5, using modified cDNA expression vectors. Karasuyama et al. Eur. J. Immunol. 18, 97-104, 1988). Since multiple copies of this vector are present within a cell as episomes, it is possible to increase the number of copies of Cre gene per cell. Furthermore, since it increases as an episome, it expresses without being affected by chromatin structure.

### (5) Mouse HCV-specific T-cell antigen peptide

When HCV specific D^{b}-binding peptides were searched for using a mouse MHC class I-binding peptide prediction program developed by Udaka et al., Kochi University (reference: An automated prediction of MHC class I-binding peptides based on positional scanning with peptide libraries. Udaka, et al. Immunogenetics. 51, 816-828, 2000.), CD649 (log Kd = -7.58) induced cytotoxic T-cells (CTL) well in the CBF1 mouse.

### (6) Whole-cell Bordetella pertussis vaccine

With regard to the Bordetella pertussis vaccine used for human beings, there are purified cell vaccine and whole-cell vaccine; among them the whole-cell vaccine is available from Bio Farma of Indonesia, and it is confirmed that if the whole-cell vaccine is administered together with a peptide, the induction of cytotoxic T-cells were augmented remarkably in mice.

### (7) Hydrodynamic injection (HDI) method

A method in which an anesthetized mouse is intravenously injected in about 5 sec with a large volume of physiological solution (about 1.5 mL) in which plasmid DNA is dissolved and the venous pressure is temporarily raised, thus increasing the efficiency of plasmid uptake. The venous pressure becomes particularly high in tissues such as the liver and spleen where venous blood is easily retained, and since part of the cell membrane is physically torn or intake an extracellular solution is accelerated due to pinocytosis, the plasmid is efficiently taken up into the cytoplasm of liver cells.

### <Hepatitis onset experiment>

A CBF1 mouse is intradermally injected with CD649 and whole-cell Bordetella pertussis vaccine once a week a total of 4 times, thus inducing cytotoxic T-cells (CTL), Cre gene is then expressed using the HDI method, and expression of the HCV gene possessed by the CBF1 mouse is induced. In this mouse, whether or not hepatitis has occurred naturally is checked by examining liver tissue and measuring serum AST and ALT enzyme (group of enzymes released from damaged liver cells) values. Furthermore, the degrees of lymphocyte infiltration and tissue damage are investigated by histological tissue examination.

### <in vivo CTL infiltration experiment>

An EGFP transgenic mouse is intradermally injected with CD649 and whole-cell Bordetella pertussis vaccine once a week a total of 4 times, thus inducing cytotoxic T-cells (CTL), and immunocytes are then taken out from the spleen and subjected to peptide stimulation twice in vitro, thus increasing the proportion of CD649-specific CTL. A CBF1 mouse in which expression of the HCV gene has been induced by the above-mentioned hepatitis onset experiment is intravenously injected with the above CTL, a few days later the liver is examined using a fluorescent microscope, and the number of EGFP positive cells that have infiltrated the liver is compared with the case of a mouse intravenously injected with an empty vector.

### <Experiment to examine in vivo tissue damage>

A vector that simultaneously expresses the Cre gene and EGFP is created and prepared so that only liver cells that induce expression of the HCV gene emit EGFP fluorescence. In the above-mentioned hepatitis onset experiment, by using this Cre-EGFP expression vector, a histology in which the liver is infiltrated by cytotoxic T-cells (CTL) and EGFP expressing cells (i.e. HCV gene expressing cells) are preferentially damaged, and the like, is observed.

## Claims

1. A method for inducing cytotoxic T-cells *in vitro,* the method comprising:
generating a target cell for cytotoxic T-cells on which surface HLA molecules are bound by an amino acid sequence consisting of SEQ ID NO: 1.

2. The method as set forth in Claim 1, wherein the peptide binds to a human HLA-A24 molecule on the surface of the target cell.

3. A cytotoxic T-cell inducer for use in a method of inducing cytotoxic T-cells, for use in a method of preventing or treating a disease that is caused by hepatitis C virus, the cytotoxic T-cell inducer comprising a peptide, the peptide consisting of an amino acid sequence consisting of SEQ ID NO: 1, wherein the method of inducing cytotoxic T-cells comprises a step of binding the peptide to an HLA molecule on the surface of a cell that is a target of a cytotoxic T-cell.

4. A pharmaceutical composition for use in a method of treating a disease that is caused by hepatitis C virus, the composition comprising a peptide, the peptide consisting of an amino acid sequence consisting of SEQ ID NO: 1, wherein the method of treating comprises a step of binding the peptide to an HLA molecule on the surface of a cell infected with hepatitis C virus.

5. A vaccine for use in a method of preventing or treating a disease that is caused by hepatitis C virus, the vaccine comprising a peptide, the peptide consisting of an amino acid sequence consisting of SEQ ID NO: 1, wherein the method comprises a step of binding the peptide to an HLA molecule on the surface of a cell infected with hepatitis C virus.

## Patentansprüche

1. Verfahren zum Induzieren zytotoxischer T-Zellen *in vitro,* wobei das Verfahren Folgendes beinhaltet:
Erzeugen einer Zielzelle für zytotoxische T-Zellen, auf deren Oberfläche HLA-Moleküle durch eine Aminosäuresequenz bestehend aus SEQ ID Nr. 1 gebunden werden.

2. Verfahren nach Anspruch 1, wobei sich das Peptid an ein humanes HLA-A24-Molekül auf der Oberfläche der Zielzelle bindet.

3. Zytotoxischer T-Zell-Inducer zur Verwendung in einem Verfahren zum Induzieren zytotoxischer T-Zellen, zur Verwendung in einem Verfahren zur Verhütung oder Behandlung einer Krankheit, die durch das Hepatitis-C-Virus verursacht wird, wobei der zytotoxische T-Zell-Inducer ein Peptid beinhaltet, wobei das Peptid aus einer Aminosäuresequenz bestehend aus SEQ ID Nr. 1 besteht, wobei das Verfahren zum Induzieren zytotoxischer T-Zellen einen Schritt zum Binden des Peptids an ein HLA-Molekül auf der Oberfläche einer Zelle beinhaltet, die ein Ziel einer zytotoxischen T-Zelle ist.

4. Pharmazeutische Zusammensetzung zur Verwendung in einem Verfahren zur Behandlung einer Krankheit, die durch das Hepatitis-C-Virus verursacht wird, wobei die Zusammensetzung ein Peptid beinhaltet, wobei das Peptid aus einer Aminosäuresequenz bestehend aus SEQ ID Nr. 1 besteht, wobei das Behandlungsverfahren einen Schritt zum Binden des Peptids an ein HLA-Molekül auf der Oberfläche einer mit dem Hepatitis-C-Virus infizierten Zelle beinhaltet.

5. Impfstoff zur Verwendung in einem Verfahren zur Verhütung oder Behandlung einer Krankheit, die durch das Hepatitis-C-Virus verursacht wird, wobei der Impfstoff ein Peptid beinhaltet, wobei das Peptid aus einer Aminosäuresequenz bestehend aus SEQ ID Nr. 1 besteht, wobei das Verfahren einen Schritt zum Binden des Peptids an ein HLA-Molekül auf der Oberfläche einer mit dem Hepatitis-C-Virus infizierten Zelle beinhaltet.

## Revendications

1. Procédé d'induction de cellules T cytotoxiques *in vitro*, le procédé comprenant :
générer une cellule cible pour des cellules T cytotoxiques sur la surface de laquelle des molécules HLA sont liées par une séquence d'acides aminés consistant en la SEQ ID NO: 1.

2. Procédé selon la revendication 1, dans lequel le peptide se lie à une molécule HLA-A24 humaine sur la surface de la cellule cible.

3. Inducteur de cellules T cytotoxiques à utiliser dans un procédé d'induction de cellules T cytotoxiques, à utiliser dans une méthode de prévention ou de traitement d'une maladie qui est causée par le virus de l'hépatite C, où l'inducteur de cellules T cytotoxiques comprend un peptide, le peptide consistant en une séquence d'acides aminés consistant en la SEQ ID NO: 1, le procédé d'induction de cellules T cytotoxiques comprenant une étape de liaison du peptide à une molécule HLA sur la surface d'une cellule qui est une cible d'une cellule T cytotoxique.

4. Composition pharmaceutique à utiliser dans une méthode de traitement d'une maladie qui est causée par le virus de l'hépatite C, la composition comprenant un peptide, le peptide consistant en une séquence d'acides aminés consistant en la SEQ ID NO: 1, la méthode de traitement comprenant une étape de liaison du peptide à une molécule HLA sur la surface d'une cellule infectée par le virus de l'hépatite C.

5. Vaccin à utiliser dans une méthode de prévention ou de traitement d'une maladie qui est causée par le virus de l'hépatite C, le vaccin comprenant un peptide, le peptide consistant en une séquence d'acides aminés consistant en la SEQ ID NO: 1, où la méthode comprend une étape de liaison du peptide à une molécule HLA sur la surface d'une cellule infectée par le virus de l'hépatite C.
